# EUROPEAN PATENT APPLICATION

(11) **EP 4 595 899 A1**
(43) Date of publication of application: **06.08.2025**
(21) Application number: 25154196.7
(22) Date of filing: 27.01.2025
(51) Int. Cl.: A61B 8/08, A61B 8/00, A61B 17/00, A61B 34/10, A61B 34/20, A61B 90/00

(54) **PROSTATE PUNCTURE SUPPORT DEVICE, METHOD OF OPERATING PROSTATE PUNCTURE SUPPORT DEVICE, AND NON-TRANSITORY COMPUTER READABLE MEDIUM**

(30) Priority: 31.01.2024 JP 2024012551
(71) Applicant: FUJI-FILM Corporation, Minato-ku Tokyo 106-8620 (JP)
(72) Inventor: GOTO, Tsubasa, Tokyo (JP); ISHIGURO, Suguru, Chiba (JP); WAKI, Koji, Chiba (JP)
(74) Representative: Dehns Germany Partnerschaft mbB

(57) **Abstract**

There are provided a prostate puncture support device, a method of operating a prostate puncture support device, and a program for a prostate puncture support device in which the degrees of freedom of a puncture position and a puncture angle are secured and the complication of the management of cables of sensors, a magnetic field fluctuation, and the like are suppressed.

A prostate puncture system (10) includes a puncture support device (15) that supports puncture during a prostate biopsy. A surgical field image acquisition unit (25) acquires a surgical field image (42) that is captured by a camera (14) positioned outside a body and includes a puncture needle image marker (13) attached to a puncture needle (12); and a position/posture information acquisition unit (21) analyzes the image marker to obtain position/posture information. A puncture information output unit (27) outputs puncture support information of the puncture needle that is based on the position/posture information obtained by the position/posture information acquisition unit, and a display control unit superimposes the puncture support information on an image to generate a superimposed image and displays the superimposed image on a display.

## Description

### BACKGROUND OF THE INVENTION

### 1. Field of the Invention

The present invention relates to a prostate puncture support device, a method of operating a prostate puncture support device, and a program for a non-transitory computer readable medium, for a case where a puncture needle is inserted into a prostate.

### 2. Description of the Related Art

There is a prostate biopsy as one of the methods of checking whether or not prostate cancer is present. A biopsy is a method that includes cutting out and collecting a part of a body and performing a pathological examination on the collected cells or tissues to determine whether or not a disease is present. In a prostate biopsy, a user inserts an ultrasound probe into a body through the rectum and inserts a thin needle (puncture needle) into a prostate to collect (puncture) a tissue while viewing the state of a prostate with an ultrasound image. It is common to collect tissues by inserting a needle into 10 or more sites in one examination.

In a prostate biopsy, since a lesion is difficult to be seen only with an ultrasound image in a case where the lesion is small, it may be difficult to accurately puncture a lesion part. In order to solve such a problem, a puncture support device has been developed at present. As the puncture support device, there is a prostate biopsy system that increases the accuracy of puncture by fusing an MRI image with an ultrasound image (MRI-US Fusion) and displaying the fused image in real time in addition to puncture support using a surgical robot (for example, MRI-US fusion prostate biopsy system (ARIETTA 65 Intuitive Fusion; ARIETTA 65 Intuitive Fusion), [online], [Searched on December 13, 2023], Internet <URL: https://www.innervision.co.jp/sp/products/release/2023 0524>).

### SUMMARY OF THE INVENTION

Since a puncture support device needs to use a template that fixes a puncture path of a puncture needle to specify a puncture position in an encoder type fusion prostate biopsy system, there is a problem that a puncture position and a puncture angle are limited. Further, since it is necessary to attach a sensor to each surgical instrument in a magnetic sensor type fusion biopsy system, the management of cables is complicated.

An object of an aspect of the invention is to provide a prostate puncture support device, a method of operating a prostate puncture support device, and a non-transitory computer readable medium in which the degrees of freedom of a puncture position and a puncture angle are secured and the complication of the management of cables of sensors is suppressed.

A prostate puncture support device according to an aspect of the invention comprises a processor. The processor acquires a reference volume image that is acquired before surgery and includes a prostate and a region of interest in the prostate, a surgical field image that is acquired by a camera positioned outside a body and includes at least a puncture needle image marker attached to a puncture needle, and an ultrasound image that is acquired by an ultrasound probe; and outputs puncture support information on the puncture needle to the ultrasound image on the basis of position/posture information of the puncture needle obtained by analyzing the puncture needle image marker.

It is preferable that the processor performs registration between the reference volume image and the ultrasound image on the basis of position/posture information of the ultrasound probe.

It is preferable that the surgical field image further includes a probe image marker attached to the ultrasound probe, and the processor acquires the position/posture information of the ultrasound probe by analyzing the probe image marker included in the surgical field image.

It is preferable that the surgical field image further includes a magnetic field generator image marker attached to a magnetic field generator, the ultrasound probe includes a magnetic sensor, and the processor calculates the position/posture information of the ultrasound probe on the basis of position/posture information obtained by the magnetic sensor of the ultrasound probe and position/posture information of the magnetic field generator image marker attached to the magnetic field generator.

It is preferable that the surgical field image further includes a subject image marker attached to a body surface of a subject, and the processor acquires position/posture information of the body surface from the subject image marker to correct misregistration between the reference volume image and the ultrasound image.

It is preferable that the processor identifies a plurality of markers including the puncture needle image marker attached to the puncture needle, the probe image marker attached to the ultrasound probe, and the subject image marker attached to the body surface, using colors and/or shapes of the image markers.

It is preferable that the processor identifies a plurality of markers including the puncture needle image marker attached to the puncture needle, the magnetic field generator image marker attached to the magnetic field generator, and the subject image marker attached to the body surface, using colors and/or shapes of the image markers.

It is preferable that the puncture support information on the puncture needle is a puncture guideline for puncture.

It is preferable that the processor superimposes the puncture support information, which includes a region of interest in the reference volume image and the puncture guideline, on the ultrasound image.

It is preferable that a region of interest in the acquired reference volume image is an entire image or a specific region such as a target organ or a peripheral image.

It is preferable that the processor superimposes the puncture support information, which includes the ultrasound image, a region of interest in the reference volume image, and the puncture guideline, on the surgical field image.

It is preferable that the ultrasound image is a 3D ultrasound image, and the processor acquires position/posture information of a 2D ultrasound image using the probe image marker attached to the ultrasound probe and generates the 3D ultrasound image on the basis of the acquired position/posture information.

It is preferable that the processor superimposes the ultrasound image, the reference volume image, and the puncture guideline on a virtual space in which a target organ is 3D-rendered.

A method of operating a prostate puncture support device according to another aspect of the invention comprises: a step of acquiring a reference volume image that is acquired before surgery and includes a prostate and a region of interest in the prostate and a surgical field image that is acquired by a camera positioned outside a body and includes at least a puncture needle image marker attached to a puncture needle; and a step of outputting puncture support information on the puncture needle on the basis of position/posture information of the puncture needle obtained by analyzing the puncture needle image marker.

A non-transitory computer readable medium storing a computer-executable program according to another aspect of the invention causes a computer to realize: a function to acquire a reference volume image that is acquired before surgery and includes a prostate and a region of interest in the prostate and a surgical field image that is acquired by a camera positioned outside a body and includes at least a puncture needle image marker attached to a puncture needle; and a function to output puncture support information on the puncture needle on the basis of position/posture information of the puncture needle obtained by analyzing the puncture needle image marker.

According to the exemplary embodiment of the invention, the degrees of freedom of a puncture position and a puncture angle can be secured and the complication of the management of cables of sensors, a magnetic field fluctuation, and the like can be suppressed.

### BRIEF DESCRIPTION OF THE DRAWINGS

Fig. 1 is a schematic diagram of a prostate puncture support system.
Fig. 2 is a block diagram showing functions of a puncture support device.
Fig. 3 is a diagram illustrating a system configuration 1 of the puncture support device.
Fig. 4 is a diagram illustrating a system configuration 2 of the puncture support device.
Fig. 5 is a diagram illustrating a system configuration 3 of the puncture support device.
Fig. 6A is a diagram illustrating the mounting of a prostate puncture support system on a patient, and Fig. 6B is a diagram illustrating a display in which positional information of a puncture needle and positional information of a biopsy are superimposed on a fusion image.
(A) of Fig. 7 is a diagram illustrating a display in which puncture support information is superimposed on a fusion image, and (B) of Fig. 7 is a diagram illustrating a display in which puncture support information is superimposed on a surgical field image.
Fig. 8 is a block diagram showing functions of a 3D image generation unit.
Fig. 9A is a diagram illustrating a display of a 3D ultrasound image, and Fig. 9B is a diagram illustrating a display in which the 3D ultrasound image and the fusion image are superimposed on a 3D-rendered image of a target organ.
Fig. 10 is a flowchart showing a series of flows related to a prostate biopsy.

### DESCRIPTION OF THE PREFERRED EMBODIMENTS

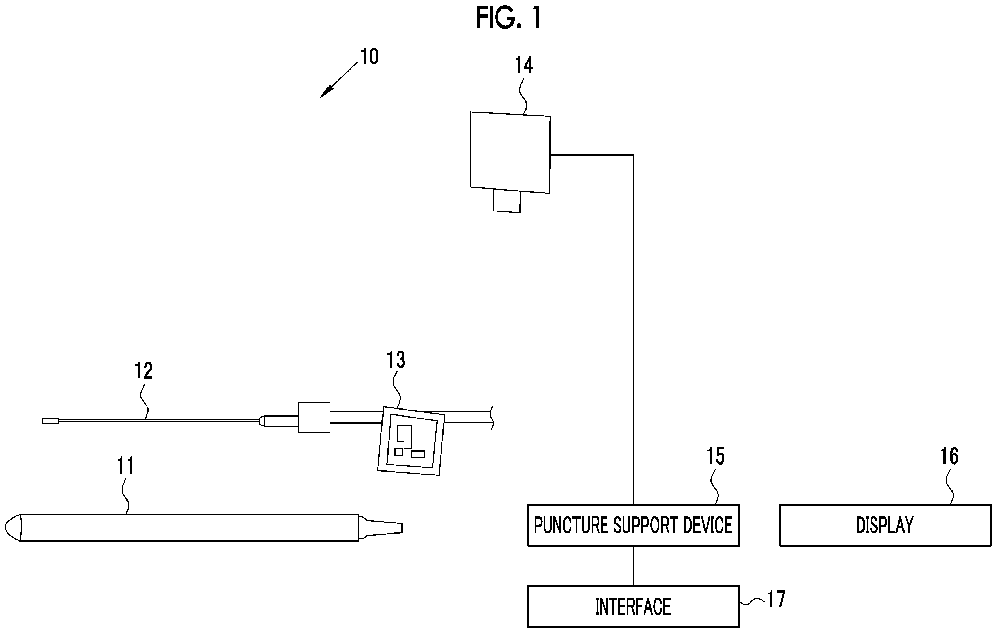

As shown in Fig. 1, a prostate puncture system 10 comprises an ultrasound probe 11, a puncture needle 12, a puncture needle image marker 13, a camera 14, a puncture support device 15, a display 16, and an interface 17. The puncture support device 15 is electrically connected to the ultrasound probe 11, the camera 14, the display 16, and the interface 17. The connection of these is not limited to wired connection and may be wireless connection. Further, connection may be performed via a network.

In the prostate puncture system 10, in order to perform a biopsy of a tissue suspected to be cancer in a prostate, a user inserts the ultrasound probe 11 into a body of a patient through the rectum to acquire an ultrasound image and collects the tissue of the prostate using the puncture needle 12 while viewing the ultrasound image. Whether or not cancer is present in the collected tissue of the prostate is pathologically checked by microscopy.

The prostate puncture system 10 comprises the puncture support device 15 that supports puncture during a prostate biopsy. The position of the puncture needle is superimposed on the ultrasound image using the puncture needle image marker 13, the camera 14, and the puncture support device 15, and is displayed on the display 16. The interface 17 includes a keyboard, a mouse, a touch pad, a microphone, a foot pedal, and the like, and has a function to receive an input operation, such as function settings.

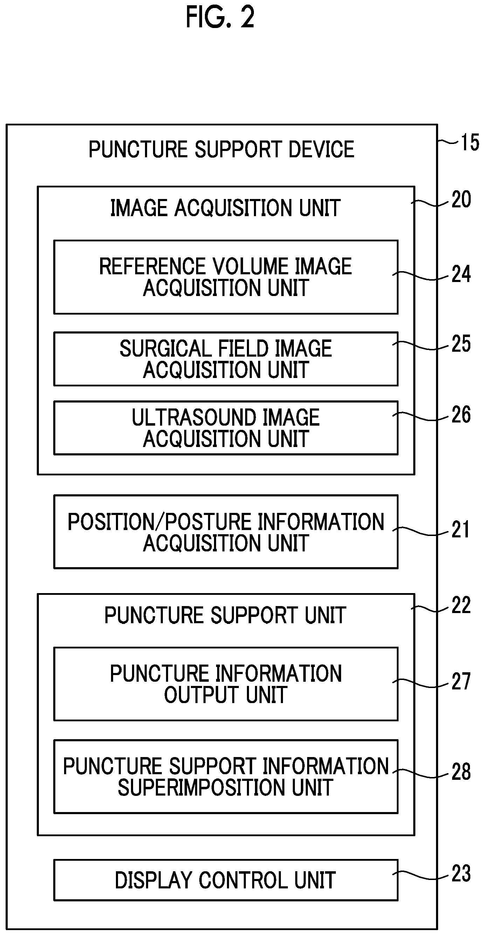

As shown in Fig. 2, the puncture support device 15 comprises an image acquisition unit 20, a position/posture information acquisition unit 21, a puncture support unit 22, and a display control unit 23. The puncture support device 15 is provided with a program memory (not shown) that stores a specific program. A control unit (not shown) forming a processor executes a specific program to realize the functions of the image acquisition unit 20, the position/posture information acquisition unit 21, the puncture support unit 22, and the display control unit 23.

The image acquisition unit 20 comprises a reference volume image acquisition unit 24, a surgical field image acquisition unit 25, and an ultrasound image acquisition unit 26. The reference volume image acquisition unit 24 acquires a reference volume image that is acquired before surgery and includes a region of interest in the prostate. The reference volume image is obtained from an image examination that is performed to predict whether or not prostate cancer is present. Further, the region of interest is a region that represents a tissue suspected to be prostate cancer. In recent years, an MRI examination has been widely used as an image examination of prostate cancer, but generates a strong magnetic field. For this reason, in a case where metal such as a pacemaker is present in a body of a patient, a CT examination using X-rays, a PET examination in which a patient is administered with an examination drug in which glucose is labeled with a radioactive isotope and radiation emitted from the examination drug is detected to acquire an image, or the like is used.

The surgical field image acquisition unit 25 acquires a surgical field image that is captured by the camera 14 positioned outside the body and includes the puncture needle image marker 13 attached to the puncture needle 12. The ultrasound image acquisition unit 26 acquires an ultrasound image that is obtained by the ultrasound probe inserted into the body of the patient through the rectum. The image is transmitted to the puncture support device 15 in a wired or wireless manner.

The position/posture information acquisition unit 21 analyzes the image marker to obtain position/posture information. The image marker may be attached not only to the puncture needle 12 but also to other surgical tools or a body surface of the patient, or may be attached to a device such as a magnetic field generator. Since there are a plurality of combinations of image markers, details thereof will be described in a system configuration to be described later.

The puncture support unit 22 comprises a puncture information output unit 27 and a puncture support information superimposition unit 28. The puncture information output unit 27 outputs puncture support information of the puncture needle 12 that is based on the position/posture information obtained by the position/posture information acquisition unit 21. The display control unit 23 performs registration between the reference volume image and the ultrasound image on the basis of position/posture information of the ultrasound probe to be described later to generate a fusion image. Further, the display control unit 23 superimposes the puncture support information output from the puncture information output unit 27 on the image acquired by the image acquisition unit 20 to generate a superimposed image, and displays the superimposed image on the display 16. Since there are a plurality of images to be superimposed, details thereof will be described in a superimposition pattern to be described below.

With regard to a method of outputting the puncture support information of the puncture needle 12 using the combinations of image markers, the following three system configurations will be described.

### System configuration 1

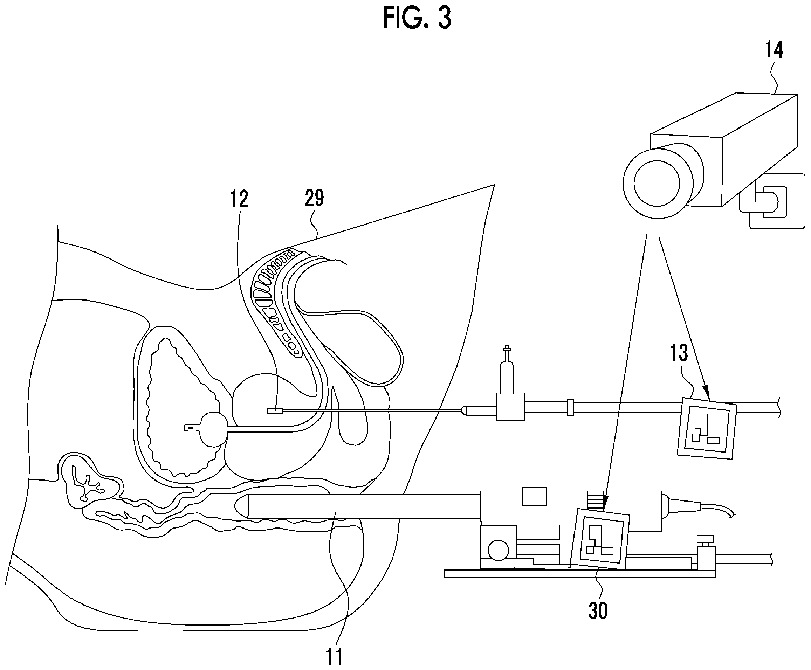

A case where the surgical field image acquisition unit 25 acquires a surgical field image including not only the puncture needle image marker 13 but also a probe image marker attached to the ultrasound probe will be described. For example, in a case where a prostate biopsy is performed on a patient 29 as shown in Fig. 3, the surgical field image acquisition unit 25 acquires a surgical field image, which includes the puncture needle image marker 13 and a probe image marker 30 attached to the ultrasound probe, with the camera 14. The position/posture information acquisition unit 21 can acquire not only the position/posture information of the puncture needle 12 but also the position/posture information of the ultrasound probe 11 on the basis of the acquired surgical field image. Each of the puncture needle image marker 13 and the probe image marker 30 is a marker with a symbol or a pattern (for example, a pattern in which a plurality of black circles and black quadrangular figures are arranged) and may be black- and-white or colorful. Further, it is preferable that the puncture needle image marker 13 and the probe image marker 30 are changed in appearance depending on an imaging angle (for example, in a case where the marker is viewed obliquely in a perspective situation, a gap between figures is seen to be narrowed as compared to a case where the marker is viewed from the front). Furthermore, each of the puncture needle image marker 13 and the probe image marker 30 may have a two-dimensional shape or a three-dimensional shape, and may be attached to a two-dimensional surface or a three-dimensional curved surface. In addition, it is preferable that three-dimensional position/posture information of the camera 14 can be acquired through the reading of the symbols or patterns of the puncture needle image marker 13 and the probe image marker 30 from an image captured by the camera 14.

According to the above-described configuration, the display control unit 23 performs registration between the reference volume image and the ultrasound image on the basis of the position/posture information of the ultrasound probe. Then, the display control unit 23 generates a fusion image using the registration. Further, a magnetic field generator to be described later and a magnetic field generator image marker attached to the magnetic field generator may be used as the position/posture information of the ultrasound probe. The magnetic field generator is used for, for example, puncture navigation using a magnetic system. The magnetic field generator is used with a puncture needle that includes a magnetic sensor built in a needle tip thereof, an ultrasound probe to which the magnetic sensor is attached, and a magnetic field generator, the position of the needle tip in which the sensor is housed and the position of the ultrasound probe can be detected through a magnetic field generated by the magnetic field generator. Furthermore, the positional information of the magnetic field generator image marker can be three-dimensionally acquired using the camera, as in the case of the puncture needle image marker.

### System configuration 2

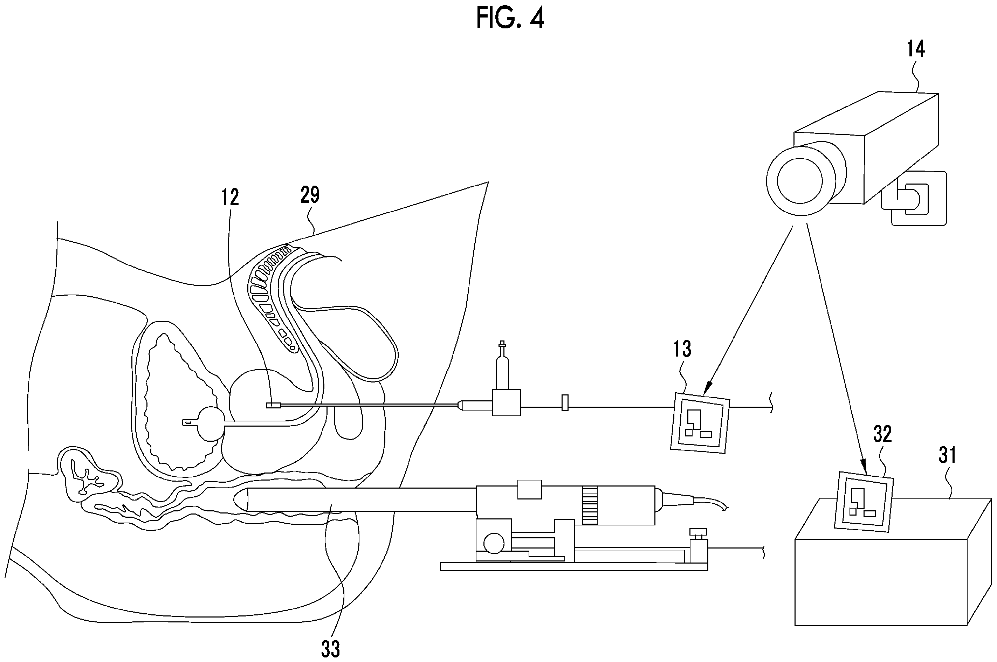

A case where the surgical field image acquisition unit 25 acquires a surgical field image including not only the puncture needle image marker 13 but also a magnetic field generator image marker attached to a magnetic field generator will be described. For example, in a case in which a prostate biopsy is performed on the patient 29 using the camera 14, a magnetic field generator 31, and an ultrasound probe 33 with a magnetic sensor as shown in Fig. 4, the surgical field image acquisition unit 25 acquires a surgical field image, which includes the puncture needle image marker 13 attached to the puncture needle 12 and a magnetic field generator image marker 32 attached to a magnetic field generator, with the camera 14. The position/posture information acquisition unit 21 acquires position/posture information on the basis of the magnetic field generator image marker 32 included in the acquired surgical field image. Further, the position/posture information acquisition unit 21 adds a position vector of the magnetic field generator 31 to a position vector of the ultrasound probe 33 with the magnetic sensor to acquire the position/posture information of the ultrasound probe with respect to the camera 14. For this reason, the position/posture information acquisition unit 21 can calculate the position/posture information of the ultrasound probe on the basis of position/posture information obtained by the magnetic sensor of the ultrasound probe and the position/posture information of the magnetic field generator image marker 32 attached to the magnetic field generator. It is preferable that the puncture needle image marker 13 and the magnetic field generator image marker 32 are AR markers.

### System configuration 3

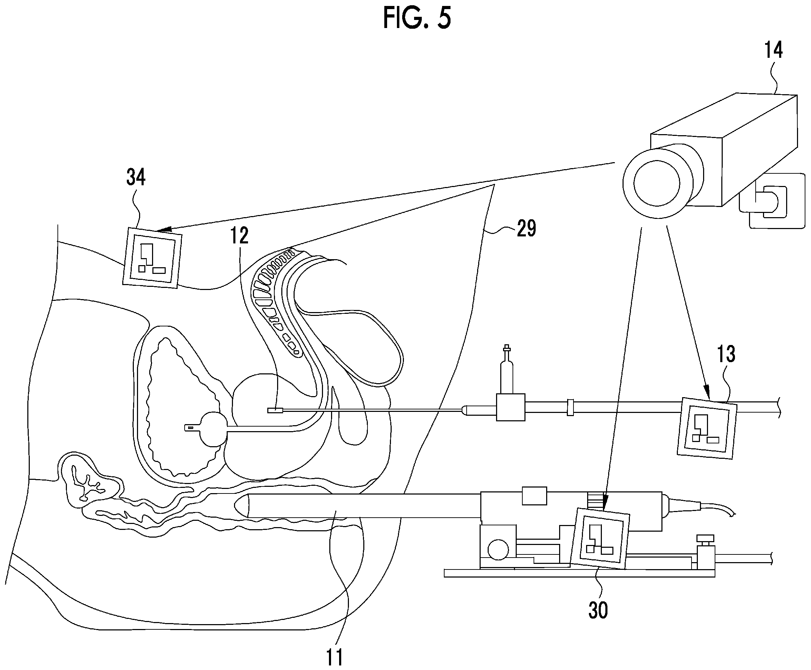

A case where the surgical field image acquisition unit 25 acquires a surgical field image including not only System configuration 1 but also a subject image marker attached to a body surface of a subject will be described. The positional information of the subject image marker can be three-dimensionally acquired using the camera, as in the case of the puncture needle image marker and the magnetic field generator image marker described above. For example, as shown in Fig. 5, in a case where a prostate biopsy is performed on the patient 29 using a subject image marker 34 in addition to System configuration 1, the surgical field image acquisition unit acquires a surgical field image, which includes the puncture needle image marker 13 attached to the puncture needle 12, the probe image marker 30 attached to the ultrasound probe 11, and the subject image marker 34 attached to the body surface of the patient 29, with the camera 14. The position/posture information acquisition unit 21 acquires the position/posture information of the body surface from the subject image marker on the basis of the acquired surgical field image. The display control unit 23 can correct misregistration between the reference volume image and the ultrasound image, which is caused by the body movement of the patient 29, in the fusion image generated using the above-described registration, on the basis of the position/posture information of the body surface. It is preferable that the subject image marker attached to the body surface of the subject is an AR marker.

In System configurations 1 to 3 described above, the position/posture information acquisition unit 21 identifies a plurality of markers including the puncture needle image marker attached to the puncture needle, the probe image marker attached to the ultrasound probe, and the subject image marker attached to the body surface, using the colors and/or shapes of the image markers. Further, the position/posture information acquisition unit 21 also identifies a plurality of markers including the magnetic field generator image marker attached to the magnetic field generator, using the colors and/or shapes of the image markers. According to the above-described configuration, it is possible to acquire the position/posture of each instrument only by attaching a marker to each instrument. Furthermore, since the position/posture information is acquired, a template that connects the puncture needle and the ultrasound probe and is used to specify a puncture position does not need to be used. As a result, a puncture position and a puncture angle are not limited, so that a puncture can be freely made.

The following three patterns will be described with regard to the superimposition of the puncture support information and an image display pattern.

### Puncture support image pattern 1

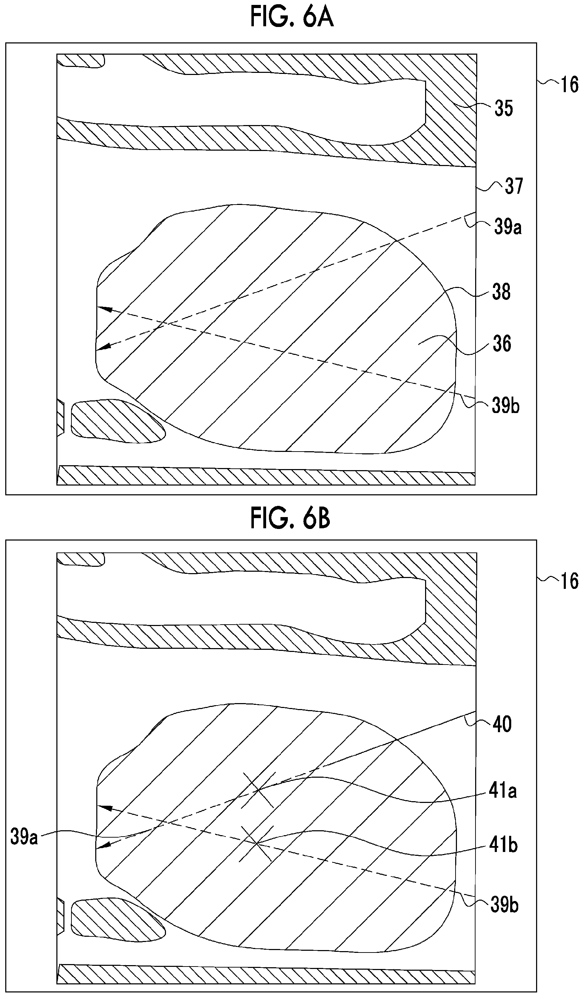

It is preferable that puncture support information on the puncture needle is a puncture guideline for puncture. The puncture support information superimposition unit 28 superimposes a region of interest in the reference volume image and the puncture support information including the puncture guideline on the ultrasound image acquired by the image acquisition unit 20. As shown in Fig. 6A, the puncture support information superimposition unit 28 superimposes puncture guidelines 39a and 39b on a region 38 of interest in the reference volume image in a fusion image 37 in which registration between an ultrasound image 35 and a reference volume image 36 is performed by the display control unit 23, and displays the superimposed image on the display 16. The region 38 of interest in the reference volume image may be the entire image, or a specific region such as a target organ or a peripheral image may be displayed as the region 38 of interest.

As shown in Fig. 6B, for example, in a case where a prostate biopsy is performed, the puncture support information superimposition unit 28 performs a control to change the display of a position on the puncture guidelines 39a and 39b, through which the puncture needle has travelled, on the display 16 into a puncture needle position 40, which is a solid line, in a case where the puncture needle 12 has punctured the prostate. Further, the puncture support information superimposition unit 28 performs a control to display biopsy positions 41a and 41b that are positions where a trigger of a biopsy gun is pulled. The display of the puncture needle position 40 and the biopsy positions 41a and 41b may be changed to any display by a user. The puncture guidelines 39a and 39b are superimposed on the fusion image 37 on the basis of a reference volume image acquired before surgery and an anatomical model of the prostate. In practice, it is preferable that not only the display of the puncture needle position 40 is changed into a solid line but also the color thereof is changed. Furthermore, although a plurality of puncture guidelines 39a and 39b are displayed, it is preferable that the puncture guidelines 39a and 39b are displayed such that the number of times of puncture is minimized.

The puncture support image pattern 1 can be implemented in any of System configurations 1 to 3 described above. Further, in System configuration 3, it is possible to correct misregistration between the reference volume image and the ultrasound image, which is caused by the body movement of the patient 29, on the basis of the subject image marker attached to the body surface of the subject. For this reason, the marker attached to each instrument is used in System configuration 3, so that it is possible to easily manage cables while tracking the body movement of the patient in real time and correcting the misregistration of the fusion image and to suppress a decrease in the accuracy of a magnetic field fluctuation caused by metal. Therefore, it is possible to stably display the fusion image and the puncture support information.

### Puncture support image pattern 2

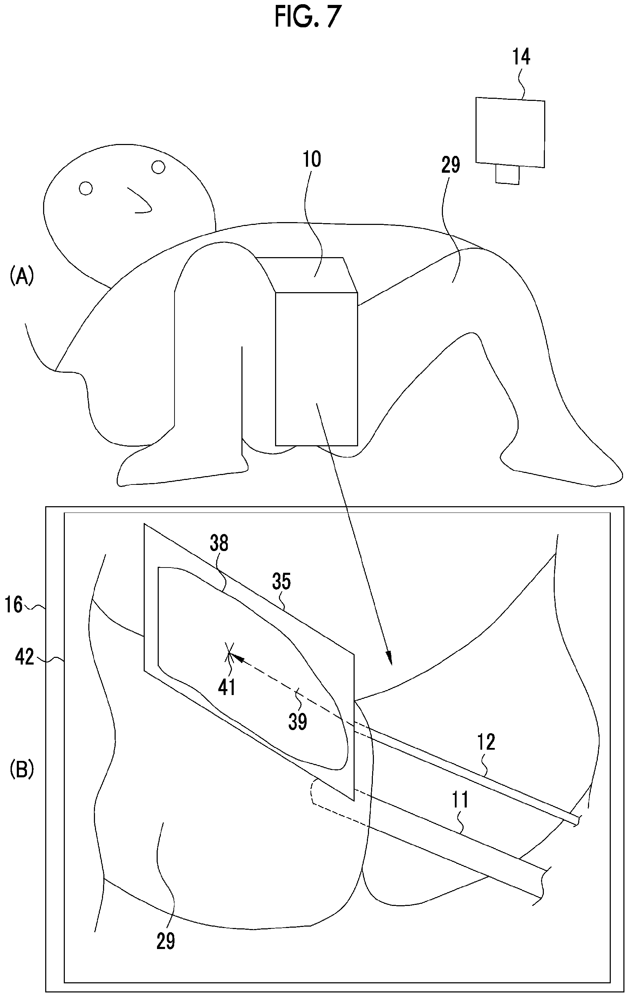

The puncture support information superimposition unit 28 may superimpose puncture support information, which includes the ultrasound image acquired by the image acquisition unit 20, the region of interest in the reference volume image, and the puncture guideline 39, on the surgical field image captured by the camera 14. For example, in a case where a prostate biopsy is performed on a patient 29 using the prostate puncture system 10 as shown in (A) of Fig. 7, the puncture support information superimposition unit 28 superimposes puncture support information, which includes an ultrasound image 35, a region 38 of interest in a reference volume image, a puncture guideline 39, and a biopsy position 41, on the surgical field image 42 in which the patient 29, the ultrasound probe 11, and the puncture needle 12 are imaged, and displays the superimposed image on the display 16 as shown in (B) of Fig. 7. For this reason, the puncture support information can show a position where the puncture needle 12 should be punctured. Further, in a case in which a user moves the puncture needle 12, the user can perform a prostate biopsy while viewing the puncture support information displayed to be superimposed on the surgical field image 42 captured by the camera 14. Furthermore, the puncture support image pattern 2 can be implemented in any of System configurations 1 to 3 described above, like the puncture support image pattern 1.

### Puncture support image pattern 3

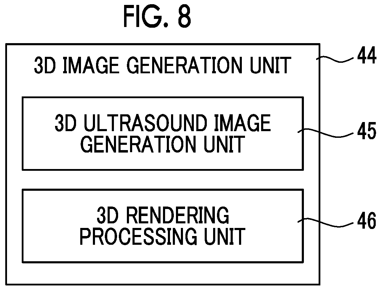

As shown in Fig. 8, the puncture support device 15 may comprise a 3D image generation unit 44. The 3D image generation unit 44 comprises a 3D ultrasound image generation unit 45 and a 3D rendering processing unit 46.

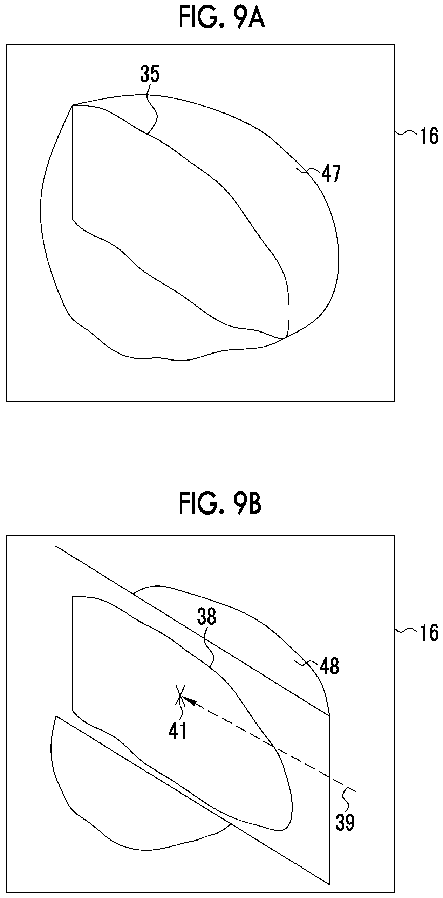

The 3D ultrasound image generation unit 45 may acquire position/posture information of a 2D ultrasound image using the probe image marker attached to the ultrasound probe and generate a 3D ultrasound image on the basis of the acquired position/posture information. For example, as shown in Fig. 9A, the position/posture information acquisition unit 21 may acquire the position/posture information of a plurality of 2D ultrasound images using a probe image marker attached to the ultrasound probe, and the 3D ultrasound image generation unit 45 may generate a 3D ultrasound image 47 on the basis of the acquired ultrasound image 35 and the acquired position/posture information and display the 3D ultrasound image 47 on the display 16. The 3D ultrasound image may be generated using a 3D ultrasound probe.

The 3D rendering processing unit 46 may superimpose an ultrasound image, a reference volume image, and a puncture guideline on a virtual space in which a target organ is 3D-rendered. For example, as shown in Fig. 9B, the 3D rendering processing unit 46 may perform 3D rendering of a target organ, and may superimpose the 3D ultrasound image generated by the 3D ultrasound image generation unit 45, the reference volume image acquired by the image acquisition unit 20, and the puncture guideline superimposed on the 3D ultrasound image by the puncture support information superimposition unit, on the virtual space in which the 3D rendering is performed. Further, the display control unit 23 may display a 3D-rendered image 48, which is superimposed on the virtual space, on the display 16. As in the puncture support image pattern 2, puncture support information includes the region 38 of interest in the reference volume image, the puncture guideline 39, and the biopsy position 41.

In the puncture support image pattern 3, a probe marker attached to the ultrasound probe or a 3D ultrasound image generated using a 3D ultrasound probe is superimposed on a virtual space in which a target organ is 3D-rendered. For this reason, in a case where a 3D ultrasound image is generated using a probe marker attached to an ultrasound probe and a plurality of 2D ultrasound images, the puncture support image pattern 3 can be implemented in any of System configuration 1 or 3 described above. Further, in a case where a 3D ultrasound image is generated using a 3D ultrasound probe, the puncture support image pattern 3 can be implemented in any of System configurations 1 to 3 described above.

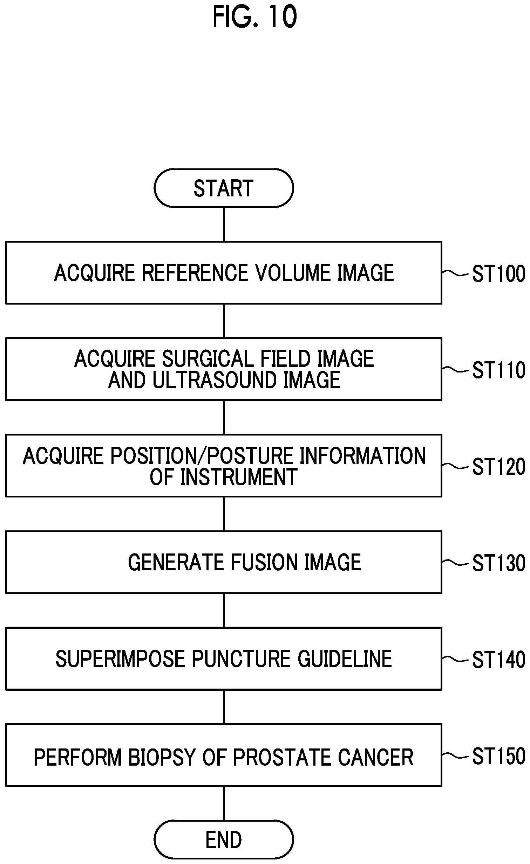

A flow of a series of processing of supporting a prostate biopsy, which is performed by the prostate puncture system 10, will be described with reference to a flowchart of Fig. 10. A user acquires a reference volume image including a region of interest suspected to be cancer before a prostate biopsy (Step ST100). In a case where a prostate biopsy is performed, a surgical field image including a marker attached to an instrument such as a puncture needle is acquired using a camera and an ultrasound image is acquired using an ultrasound probe (Step ST110). The marker appearing in the surgical field image is analyzed to acquire position/posture information of the instrument (Step ST120), and registration between a reference volume image acquired before surgery and the ultrasound image is performed to generate a fusion image (Step ST130). A puncture guideline is superimposed on the fusion image on the basis of the acquired ultrasound image and the acquired position/posture information, and the superimposed fusion image is displayed on a display (Step ST140). While observing the puncture guideline, a user operates the puncture needle to perform a biopsy of prostate cancer (Step ST150).

According to the above-described configuration of the prostate puncture system 10, a user can freely move the puncture needle while referring to the puncture support information on the virtual space in which a target organ is 3D-rendered. Therefore, a prostate biopsy can be performed with high accuracy. Further, according to the prostate puncture system 10, since the position/posture information of each surgical instrument is acquired using a marker, a template that connects the puncture needle and the ultrasound probe and fixes the position of the puncture needle does not need to be used. Therefore, the degrees of freedom of a puncture position and a puncture angle are secured and the complication of the management of cables of sensors, a magnetic field fluctuation, and the like are suppressed, so that a user can make a puncture more freely.

In the above-described embodiment, the hardware structures of processing units, which perform various types of processing, such as the image acquisition unit 20, the position/posture information acquisition unit 21, the puncture support unit 22, the display control unit 23, and the 3D image generation unit 44, are various processors to be described below. The various processors include: a central processing unit (CPU) that is a general-purpose processor functioning as various processing units by executing software (programs); a graphical processing unit (GPU); a programmable logic device (PLD) that is a processor of which the circuit configuration can be changed after manufacture, such as a field programmable gate array (FPGA); a dedicated electrical circuit that is a processor having a circuit configuration designed exclusively to perform various types of processing; and the like.

One processing unit may be formed of one of these various processors, or may be formed of a combination of two or more processors of the same type or different types (for example, a plurality of FPGAs, a combination of a CPU and an FPGA, a combination of a CPU and a GPU, or the like). Further, a plurality of processing units may be formed of one processor. As an example where a plurality of processing units are formed of one processor, first, there is an aspect in which one processor is formed of a combination of one or more CPUs and software as typified by a computer, such as a client or a server, and functions as a plurality of processing units. Second, there is an aspect in which a processor fulfilling the functions of the entire system, which includes a plurality of processing units, using one integrated circuit (IC) chip as typified by system on chip (SoC) or the like is used. In this way, various processing units are formed using one or more of the above-mentioned various processors as hardware structures.

In addition, the hardware structures of these various processors are more specifically electrical circuitry where circuit elements, such as semiconductor elements, are combined. Further, the hardware structure of the storage unit is a storage device, such as a hard disc drive (HDD) or a solid state drive (SSD).

### Explanation of References

10: prostate puncture system
11: ultrasound probe
12: puncture needle
13: puncture needle image marker
14: camera
15: puncture support device
16: display
17: interface
20: image acquisition unit
21: position/posture information acquisition unit
22: puncture support unit
23: display control unit
24: reference volume image acquisition unit
25: surgical field image acquisition unit
26: ultrasound image acquisition unit
27: puncture information output unit
28: puncture support information superimposition unit
29: patient
30: probe image marker
31: magnetic field generator
32: magnetic field generator image marker
33: ultrasound probe with magnetic sensor
34: subject image marker
35: ultrasound image
36: reference volume image
37: fusion image
38: region of interest
39: puncture guideline
40: puncture needle position
41: biopsy position
42: surgical field image
44: 3D image generation unit
45: 3D ultrasound image generation unit
46: 3D rendering processing unit
47: 3D ultrasound image
48: 3D-rendered image
ST100 to ST150: step

## Claims

1. A prostate puncture support device comprising:
a processor,
wherein the processor is configured to:
acquire a reference volume image (36) that is obtained before surgery and includes a prostate and a region of interest (38) in the prostate, a surgical field image (42) that is acquired by a camera (14) positioned outside a body and includes at least a puncture needle image marker (13) attached to a puncture needle (12), and an ultrasound image (35) that is acquired by an ultrasound probe (11); and
output puncture support information on the puncture needle (12) to the ultrasound image (35) on the basis of position/posture information of the puncture needle (12) obtained by analyzing the puncture needle image marker (13).

2. The prostate puncture support device according to claim 1,
wherein the processor is configured to perform registration between the reference volume image (36) and the ultrasound image (35) on the basis of position/posture information of the ultrasound probe (11).

3. The prostate puncture support device according to claim 2,
wherein the surgical field image (42) further includes a probe image marker (30) attached to the ultrasound probe (11), and
the processor is configured to acquire the position/posture information of the ultrasound probe (11) by analyzing the probe image marker (30) included in the surgical field image (42).

4. The prostate puncture support device according to claim 2,
wherein the surgical field image (42) further includes a magnetic field generator image marker (32) attached to a magnetic field generator (31),
the ultrasound probe (11) includes a magnetic sensor, and
the processor is configured to calculate the position/posture information of the ultrasound probe (11) on the basis of position/posture information obtained by the magnetic sensor of the ultrasound probe (11) and position/posture information of the magnetic field generator image marker (32) attached to the magnetic field generator (31).

5. The prostate puncture support device according to claim 3 or 4,
wherein the surgical field image (42) further includes a subject image marker (34) attached to a body surface of a subject, and
the processor is configured to acquire position/posture information of the body surface from the subject image marker (34) to correct misregistration between the reference volume image (36) and the ultrasound image (35).

6. The prostate puncture support device according to claim 5,
wherein the processor is configured to identify a plurality of markers including the puncture needle image marker (13) attached to the puncture needle (12), the probe image marker (30) attached to the ultrasound probe (11), and the subject image marker (34) attached to the body surface, using colors and/or shapes of the image markers.

7. The prostate puncture support device according to claim 5,
wherein the processor is configured to identify a plurality of markers including the puncture needle image marker (13) attached to the puncture needle (12), the magnetic field generator image marker (32) attached to the magnetic field generator (31), and the subject image marker (34) attached to the body surface, using colors and/or shapes of the image markers.

8. The prostate puncture support device according to any preceding claim,
wherein the puncture support information for the puncture needle (12) is a puncture guideline (39).

9. The prostate puncture support device according to claim 8,
wherein the processor is configured to superimpose the puncture support information, which includes a region of interest (38) in the reference volume image (36) and the puncture guideline (39), on the ultrasound image (35).

10. The prostate puncture support device according to any preceding claim,
wherein a region of interest (38) in the acquired reference volume image (36) is an entire image or a specific region, such as a target organ or a peripheral region.

11. The prostate puncture support device according to claim 8,
wherein the processor is configured to superimpose the puncture support information, which includes the ultrasound image, a region of interest (38) in the reference volume image (36), and the puncture guideline (39), on the surgical field image (42).

12. The prostate puncture support device according to claim 3,
wherein the ultrasound image (35) is a 3D ultrasound image, and
the processor is configured to acquire position/posture information of a 2D ultrasound image using the probe image marker (30) attached to the ultrasound probe (11) and generate the 3D ultrasound image on the basis of the acquired position/posture information.

13. The prostate puncture support device according to claim 8,
wherein the processor is configured to superimpose the ultrasound image (35), the reference volume image (36), and the puncture guideline (39) on a virtual space in which a target organ is 3D-rendered.

14. A method of operating a prostate puncture support device, the method comprising:
acquiring a reference volume image (36) that is obtained before surgery and includes a prostate and a region of interest (38) in the prostate and a surgical field image (42) that is acquired by a camera (14) positioned outside a body and includes at least a puncture needle image marker (13) attached to a puncture needle (12); and
outputting puncture support information on the puncture needle (12) on the basis of position/posture information of the puncture needle (12) obtained by analyzing the puncture needle image marker (13).

15. A non-transitory computer readable medium for storing a computer-executable program, the computer-executable program causing a computer to realize:
a function to acquire a reference volume image (36) that is obtained before surgery and includes a prostate and a region of interest (38) in the prostate and a surgical field image (42) that is acquired by a camera (14) positioned outside a body and includes at least a puncture needle image marker (13) attached to a puncture needle (12); and
a function to output puncture support information on the puncture needle on the basis of position/posture information of the puncture needle (12) obtained by analyzing the puncture needle image marker (13).
